# EUROPEAN PATENT SPECIFICATION

(11) **EP 1 927 313 B1**
(45) Date of publication and mention of the grant of the patent: **31.10.2012**
(21) Application number: 06797979.9
(22) Date of filing: 14.09.2006
(51) Int. Cl.: A61B 1/00, A61B 1/005

(54) **ENDOSCOPE DEVICE**
ENDOSKOPVORRICHTUNG
DISPOSITIF ENDOSCOPIQUE

(30) Priority: 22.09.2005 JP 2005276626
(43) Date of publication of application: 04.06.2008
(73) Proprietor: OLYMPUS MEDICAL SYSTEMS CORP., Tokyo 151-0072 (JP)
(72) Inventor: ONODA, Fumiyuki, Tokyo 151-0072 (JP); ODA, Tomohiko, Tokyo 151-0072 (JP); NIWA, Hiroshi, Tokyo 151-0072 (JP); MIYAKE, Kensuke, Tokyo 151-0072 (JP); SATO, Minoru, Tokyo 151-0072 (JP); AIZAWA, Chieko, Tokyo 151-0072 (JP); MIYOSHI, Yoshitaka, Tokyo 151-0072 (JP)
(74) Representative: von Hellfeld, Axel
(86) International application number: PCT/JP2006/318267
(87) International publication number: WO 2007/034732

(56) References cited:
- WO-A1-2005/082227
- JP-A- 63 264 046
- JP-A- 2002 034 912
- JP-A- 2003 052 611
- JP-A- 2004 337 311
- JP-A- 2005 237 820
- JP-A- 2005 237 820
- US-A- 5 060 632
- US-A1- 2002 169 361
- US-A1- 2002 188 174

## Description

### Technical Field

The present invention relates to an endoscope apparatus that has a device that performs signal processing of image pickup signals of an endoscope that is inserted into a body cavity or the like, and a device that calculates an insertion shape of the endoscope.

### Background Art

An endoscope that has an image pickup device built inside an insertion portion is widely used for examining the inside of a body cavity or for treatment using a treatment instrument.

In the case of an endoscope having a flexible insertion portion with a built-in image pickup device, a universal cable is extended from an operation portion provided at the rear end side of the insertion portion, and a connector provided at the end thereof is connected to an endoscope peripheral device. In a conventional endoscope system, connector receivers that are suited to the respective connector of the endoscope are provided in the endoscope peripheral device.

In this instance, in the conventional case, it has been necessary to connect the connector to a light source, and also connect an electrical connector that is connected to an image pickup device to a signal processing device.

Therefore, Japanese Utility Model Registration No. 2585832 discloses an apparatus which enables connection at one touch by insertion of an adaptor device with respect to an apparatus in which a light source and a signal processing device are integrated.

JP 2005/237820 discloses a capsule type endoscope provided at a distal end of an insertion tube comprising an air-water feeding pipeline. An AWS unit is provided with a scope connector for receiving a connector of the capsule type endoscope, such that the air-water pipeline is connected to the air-water pipeline of the AWS unit, and such that an electrical connection portion is connected to an electrical connection portion of the AWS unit.

Meanwhile, in recent years an insertion-shape calculating device has been developed in which a plurality of source coils are disposed along an insertion axis as magnetic field generating means inside the insertion portion of the endoscope. The insertion-shape calculating device calculates the insertion shape of the endoscope by detecting a magnetic field generated by the source coils using an externally provided sensing coil.

However, since conventionally a video processor that performs signal processing of image pickup signals of an endoscope and the aforementioned insertion-shape calculating device are independent devices, the endoscope must be respectively connected to the video processor and the insertion-shape calculating device, and there is a problem that the setting of the apparatus becomes complicated.

Further, with respect to the adaptor device in the case of a configuration according to the conventional example, although a devise has been disclosed that can be connected at one touch when an electrical connector and a light guide connector for a light source are built inside a signal processing device inside a light source, the adaptor device does not support a case in which the configuration comprises a conduit system.

More specifically, it is necessary to provide an air/water supply conduit in the endoscope to secure the observation function, and although it is desirable to also connect the connection of an electrical connector with one touch along with the connector of a conduit system in correspondence with a case comprising a connector of this kind of conduit system, this type of configuration is not implemented in the conventional cases.

Further, although it is also preferable the connection is possible even in a case in which a device to which a connector of the conduit system is connected and a device to which an electrical connector is connected are separate elements, this type of configuration is not implemented in the conventional cases.

The present invention has been made in view of the above described points, and an object of this invention is to provide an endoscope apparatus in which a processing system that performs signal processing of image pickup signals of an electronic endoscope and a processing system that calculates an insertion shape of the endoscope are integrated, and which can facilitate an operation to connect an electronic endoscope and a signal processing device.

Another object of the present invention is to provide an endoscope apparatus that can support as a matter of course a case of a connector of an endoscope comprising only either one of an electrical connector and a conduit connector, and that can also support a case of a connector of an endoscope comprising both thereof.

A further object of the present invention is to provide an endoscope apparatus that can support a case in which a device to which a connector of a conduit system is connected and a device to which an electrical connector is connected are separate members and, as a matter of course, a case of a connector of an endoscope comprising only either one of an electrical connector and a conduit connector, and also a case of a connector of an endoscope comprising both thereof.

### Disclosure of Invention

### Means for Solving the Problem

An endoscope apparatus according to claim 1 is provided. The endoscope apparatus comprises an electronic endoscope having a shape information generating section for generating insertion shape information inside an insertion portion, and an image pickup section that picks up images inside a body cavity; and a signal processing device integrally formed of an endoscope signal processing section that drives the electronic endoscope and processes image pickup signals from the electronic endoscope, and an endoscope insertion shape calculating section that calculates an insertion shape of the electronic endoscope based on the insertion shape information from the shape information generating section.

### Brief Description of the Drawings

Fig. 1 is an overall configuration diagram of an endoscope system according to an embodiment of the present invention;
Fig. 2 is a view showing the internal configuration of a first endoscope;
Fig. 3A is a side view showing the specific exterior shape of the first endoscope when the vicinity of an operation portion of the endoscope is viewed from the side;
Fig. 3B is a front view as viewed from the right side in Fig. 3A;
Fig. 3C is a back view as viewed from the left side in Fig. 3A;
Fig. 3D is a plan view as viewed from the top in Fig. 3A;
Fig. 4 is a view illustrating a configuration in which scope connectors are connectable to an AWS unit and an endoscope controlling system;
Fig. 5 is a block diagram showing the configuration of an electrical system in the first endoscope; and
Fig. 6 is a view showing scope connectors that are connectable to an AWS adaptor.

### Best Mode for Carrying Out the Invention

Hereunder, an embodiment of the present invention is described while referring to the drawings.

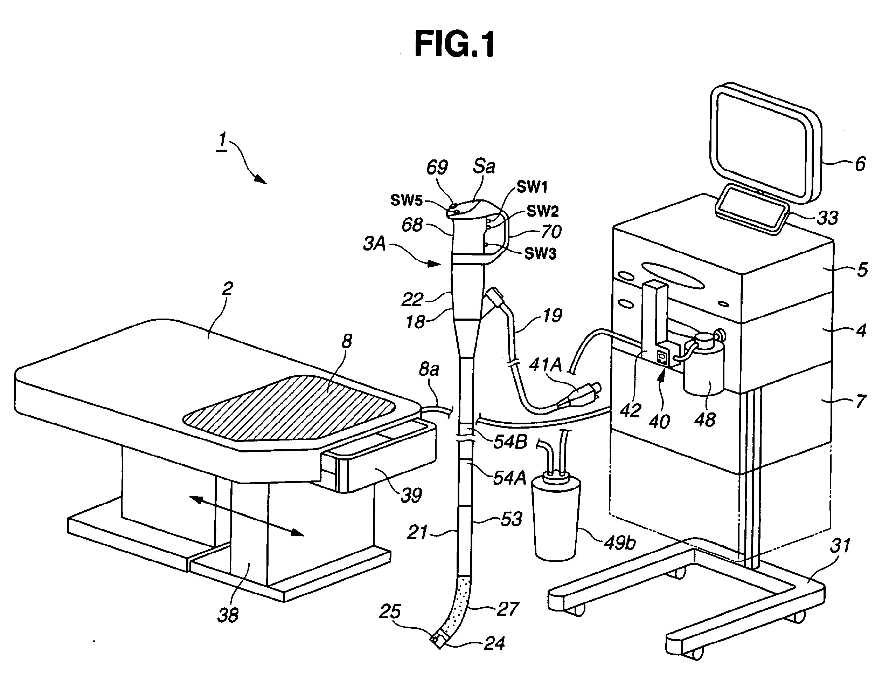

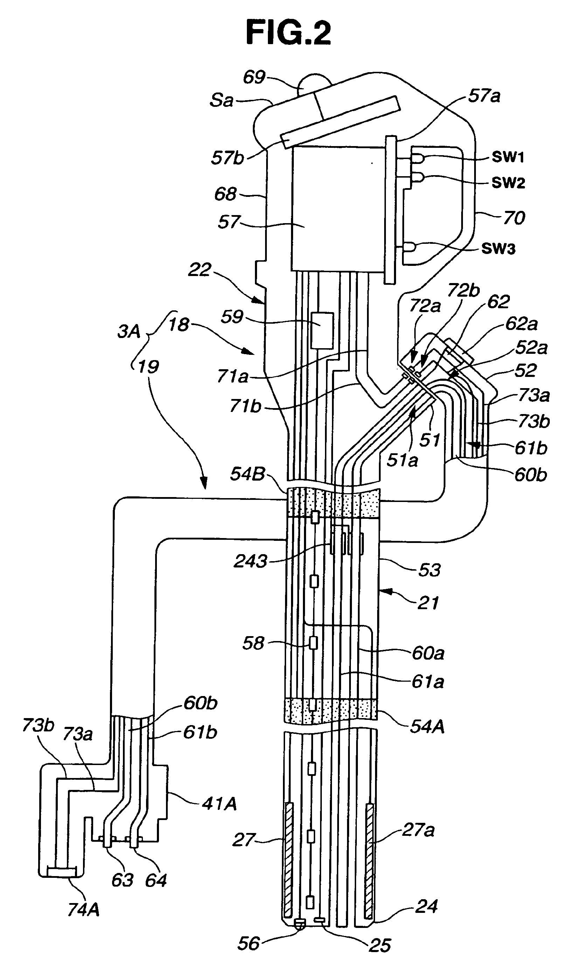

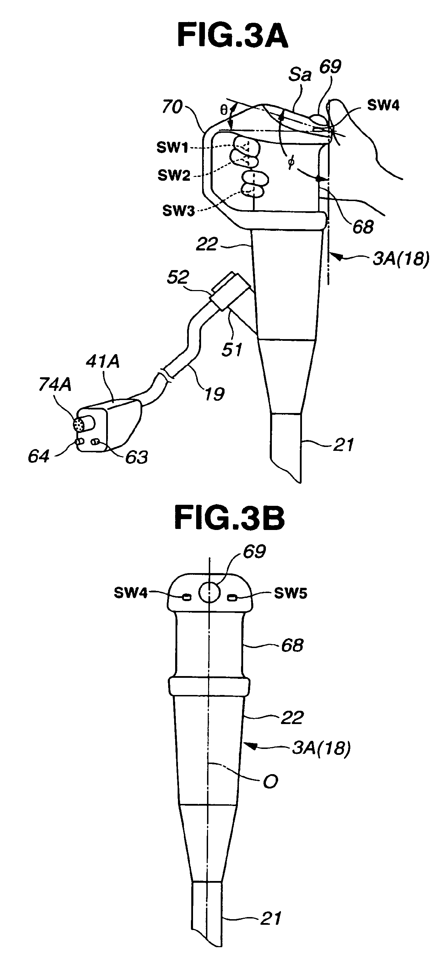

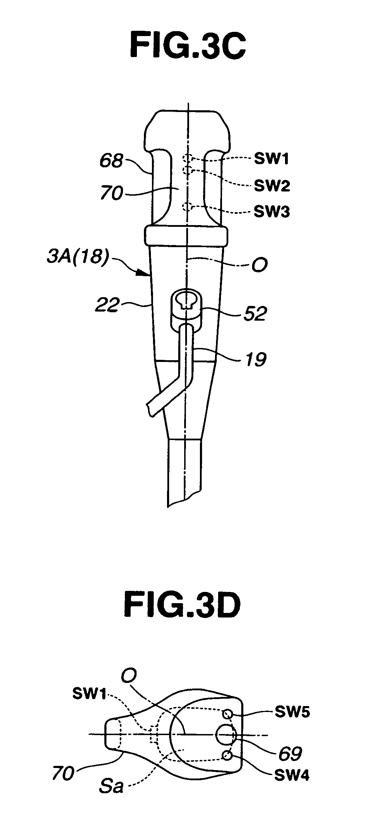

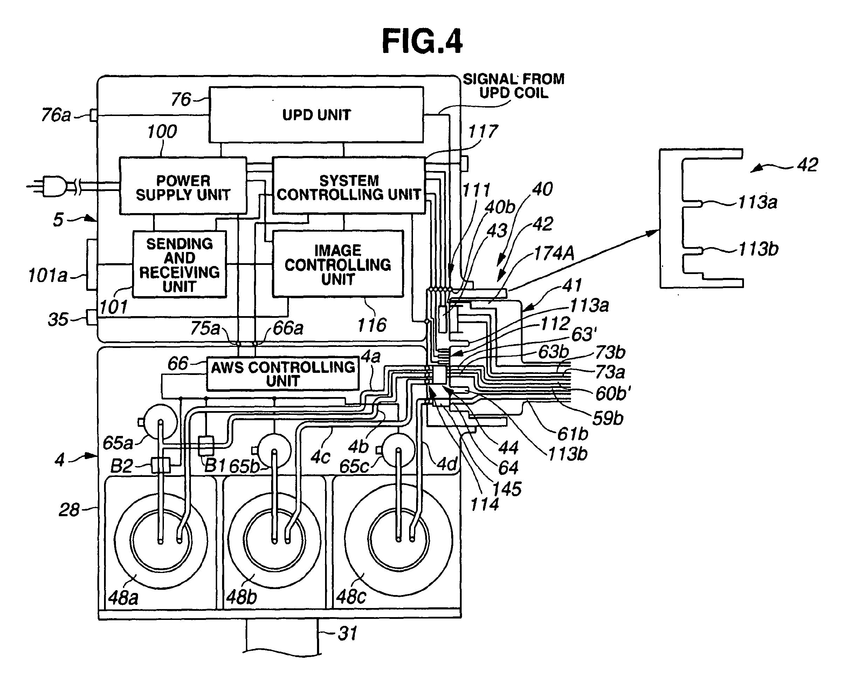

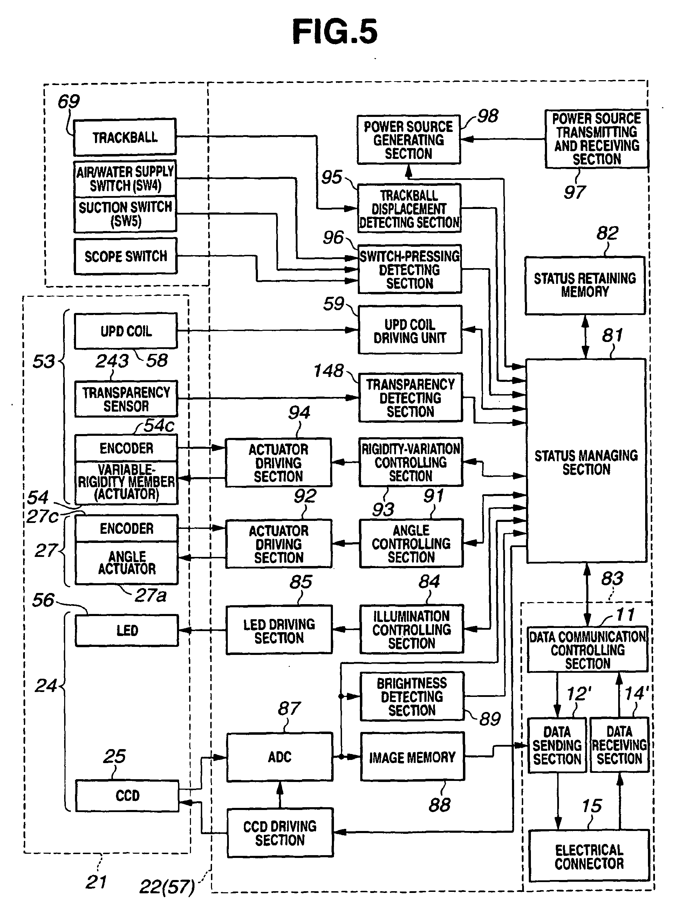

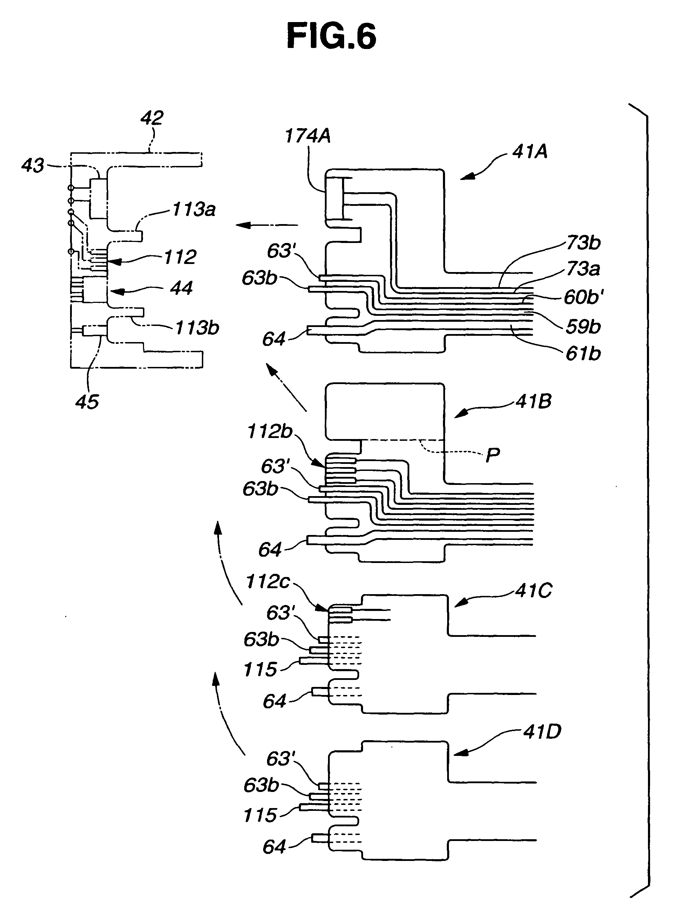

Fig. 1 to Fig. 6 relate to an embodiment of the present invention. Fig. 1 is an overall configuration diagram of an endoscope system. Fig. 2 is a view showing the internal configuration of a first endoscope. Fig. 3 includes views showing the specific exterior shape of the first endoscope. Fig. 4 is a view illustrating a configuration in which scope connectors are connectable to an AWS unit and an endoscope controlling system. Fig. 5 is a block diagram showing the configuration of an electrical system in the first endoscope. Fig. 6 is a view showing scope connectors that are connectable to an AWS adaptor.

As shown in Fig. 1, an endoscope system 1 according to an embodiment of the present invention comprises: flexible endoscopes (also referred to as scopes) 3A, 3B, and 3C (in Fig. 1, only the endoscope 3A is shown) with respectively different functions for performing endoscopy that are inserted into a body cavity of a patient (not shown) lying on an inspection bed 2; an air/water supply and suction unit (hereinafter abbreviated as "AWS unit") 4 that is detachably connected with the endoscopes 3A, 3B, and 3C and having air/water supply and suction control functions; an endoscope system controlling device 5 that performs signal processing for an image-pickup device built into the endoscopes 3A, 3B, and 3C, control processing for various operation means provided in the endoscopes 3A, 3B, and 3C, and video processing and the like; and an observation monitor 6 comprising a liquid crystal monitor or the like that displays an image signal generated by the endoscope system controlling device 5. The observation monitor 6 is provided with a touch panel 33.

The endoscope system 1 also comprises: an image recording unit 7 for performing filing and the like of, for example, a digital video signal generated by the endoscope system controlling device 5; and a UPD coil unit 8 which is connected to the AWS unit 4, and which, when shape detecting coils (hereinafter abbreviated as "UPD coils") are built inside the insertion portion of the endoscopes 3I (I = A, B, C), detects the position of each of the UPD coils by, for example, receiving an electromagnetic field that is generated by the UPD coil in question to display the shape of the insertion portion of the endoscope 3.

In the example shown in Fig. 1, the UPD coil unit 8 is arranged so as to be embedded in the top surface of the inspection bed 2. The UPD coil unit 8 is connected to the AWS unit 4 by a cable 8a.

In the present embodiment, at a position at one end in the longitudinal direction of the inspection bed 2 and beneath the inspection bed 2 is formed an accommodating concave portion is formed, in which a tray carrying trolley 38 can be accommodated. On top of the tray carrying trolley 38, a scope tray 39 for accommodating the endoscopes 31 (I = A, B, C) is mounted.

Thus, the scope tray 39 accommodating the endoscopes 3I which are sterilized or disinfected can be carried by the tray carrying trolley 38 and accommodated in the accommodating concave portion of the inspection bed 2. A surgeon can pull out the endoscopes 3I from the scope tray 39 to use for endoscopy, and may reaccommodate the endoscopes 3I in the scope tray 39 after completion of the endoscopy. Thereafter, the scope tray 39 accommodating the used endoscopes 3I can be carried by the tray carrying trolley 38 to also enable smooth sterilization or disinfection of the used endoscopes 3I.

In Fig. 1, the endoscopes 3I are detachably connected to the AWS unit 4 through scope connectors 41I (I = A, B, C, D) that are provided at an end of the tube unit 19.

Further, as shown in Fig. 1, the endoscope 3I (in this case, 1 = A) comprises an endoscope main unit 18 and a tube unit 19 that is detachably connected to the endoscope main unit 18 and is, for example, a disposable type.

The endoscope main unit 18 has an elongate flexible insertion portion 21 to be inserted into a body cavity, and an operation portion 22 that is provided at the rear end of the insertion portion 21. The proximal end of the tube unit 19 is detachably connected to the operation portion 22.

At a distal end portion 24 of the insertion portion 21 is disposed as an image pickup device, which is an image pickup section that picks up images inside a body cavity, an image pickup unit that uses a charge coupled device (abbreviated as "CCD") 25 capable of varying gain inside the image pickup device.

At the rear end of the distal end portion 24 is provided a bending portion 27 that can be made to bend with a low amount of force. By operating a trackball 69 as operation means (instruction input section) provided in the operation portion 22, the bending portion 27 can be bent. The trackball 69 is also used when performing an angle operation (bending operation) and when changing the settings of other functions of the scope switches, for example, setting the angle sensitivity or the amount of supplied air or the like.

At a plurality of locations in the insertion portion 21 are formed rigidity variation sections provided with variable-rigidity actuators 54A and 54B that are capable of changing rigidity so as to carry out an insertion operation or the like more smoothly.

The observation monitor 6 is connected to a monitor connector 35 of the endoscope system controlling device 5 by a monitor cable (see Fig. 4).

The endoscope 3A has UPD coils 58 built therein (see Fig. 2). In this case, along with image data that is picked up by the CCD 25, image data of the insertion portion shape (UPD image) of the endoscope 3A that is detected using the UPD coil unit 8 is also sent to the endoscope system controlling device 5. Accordingly, the endoscope system controlling device 5 as a signal processing device sends video signals corresponding to these types of image data to the observation monitor 6 to also display a UPD image on the display screen thereof in addition to an endoscopic image.

The observation monitor 6 is configured with a monitor of a high-definition TV (HDTV) so that a plurality of kinds of images can thus be displayed on the screen thereof at the same time.

Further, as shown in Fig. 1, for example in the endoscope system controlling device 5 and the AWS unit 4 is provided a scope connection connector 40 that corresponds to three kinds of scope connectors 41I and can be detachably connected to any of the scope connectors 41I (in Fig. 1, I = A). An adaptor 42 that can detachably attach to the scope connector 41I can be mounted on the scope connection connector 40.

In fact, on the front surface of the endoscope system controlling device 5 and the AWS unit 4, an adaptor attaching portion (not shown) in a concave shape is provided. The adaptor attaching portion is attached with an adaptor 42 to form the scope connection connector 40. The scope connector 41I on the endoscopes 3I side is connected to the scope connection connector 40.

Although not shown in the drawings, the adaptor attaching portion is provided with an electrical connector for scope connection, an air supply connector, and a pinch valve. An inner end surface of the adaptor 42 is detachably attached to the adaptor attaching portion. From an outer end surface of the adaptor 42, the scope connectors 41I of the endoscopes 3I are connected to the adaptor 42. The adaptor 42 has a conduit connection portion and an electrical connection portion that respectively connect in a detachable condition the conduit connector and the electrical connector on the endoscopes 3I side with a conduit connector and an electrical connector on the endoscope system controlling device 5 and AWS unit 4 side as endoscope peripheral devices.

Next, the specific configuration of the first endoscope 3A will be described referring to Fig. 2 and Fig. 3A to Fig. 3D. Fig. 3A is a view showing a state when the vicinity of the operation portion of the endoscope 3 is viewed from the side. Fig. 3B is a front view as seen from the right side in Fig. 3A. Fig. 3C is a back view as seen from the left side in Fig. 3A. Fig. 3D is a plan view as seen from the top in Fig. 3A.

In Fig. 1, as described in the overview thereof, the flexible endoscope 3A comprises the endoscope main unit 18 having an elongate flexible insertion portion 21 and an operation portion 22 that is provided at the rear end of the insertion portion 21, and the tube unit 19 of a disposable type that is detachably connected to a general connector portion 52 at a proximal end of a connector portion 51 (for tube unit connection) (see Fig. 2) that is provided in the vicinity of the proximal end (front end) of the operation portion 22 in the endoscope main unit 18.

At a distal end of the tube unit 19 is provided the above-mentioned scope connector 41A that is detachably connected to the AWS unit 4.

The insertion portion 21 comprises the hard distal end portion 24 provided at the distal end of the insertion portion 21, the bendable bending portion 27 provided at the rear end of the distal end portion 24; and an elongated, flexible portion (hose portion) 53 extending from a rear end of the bending portion 27 to the operation portion 22. At multiple locations in the middle of the flexible portion 53, specifically at two locations, there are provided variable-rigidity actuators 54A and 54B formed of an electroconductive polymer artificial muscle (abbreviated as EPAM) which can be expanded and contracted and also changed in hardness by applying a voltage.

Inside an illumination window provided at the distal end portion 24 of the insertion portion 21, there is attached, for example, a light emitting diode (abbreviated as LED) 56 as illumination means. The illumination light of the LED 56 is projected forwardly through an illumination lens integrally attached to the LED 56 to illuminate an object to be observed such as a diseased part. The light emitting element forming the illumination means is not limited to the LED 56, and may be formed using a LD (laser diode) and the like.

Further, an observation window provided adjacent to the illumination widow is attached with an object lens (not shown). The CCD 25 incorporating a variable gain function is disposed at that image forming position, thereby forming image pickup means for picking up the image of an object.

A plurality of signal lines which are passed through the inside of the insertion portion 21 with one end connected to the LED 56 and the other end connected to the CCD 25, respectively, are connected to a control circuit 57 which is provided inside the operation portion 22 and performs intensive control processing (collective control processing).

In the above described insertion portion 21 there are disposed a plurality of UPD coils 58 at predetermined spaces along the longitudinal direction. The plurality of UPD coils 58 constitute a shape information generating section generating insertion shape information. The signal line connected to each UPD coil 58 is connected to the control circuit 57 via a UPD coil driving unit 59 provided in the operation portion 22.

At four locations in the circumferential direction of the inner wall of the casing of the bending portion 27 there are disposed angular actuators 27a as angle elements (bend elements) formed by disposing EPAMs in the longitudinal direction thereof. The angular actuator 27a and the variable-rigidity actuators 54A and 54B are also connected to the control circuit 57 via respective signal lines. The control circuit 57 is configured by, for example, implementing electronic circuit elements on a switch board 57a and a trackball board 57b.

The EPAM used for the angular actuator 27a and the variable-rigidity actuators 54A and 54B can be contracted in the thickness direction and expanded in the longitudinal direction by, for example, attaching electrodes on both surfaces of a plate-shaped EPAM and applying a voltage thereto. Further, this EPAM can change its strain amount, for example, in proportion to approximately square of the voltage applied.

When used as the angular actuator 27a, the EPAM may be formed into a wire shape to expand one side thereof and contract the other side to thereby bend the bending portion 27 in a similar manner as a function achieved by the normal wire. Further, such expansion or contraction makes it possible to vary the rigidity thereof, and such a function is utilized in the variable-rigidity actuators 54A and 54B to make the rigidity of the portion variable.

An air/water supply conduit 60a and a suction conduit 61 a are passed through the inside of the insertion portion 21, and the rear end thereof forms a conduit connector 51a which opens at the connector portion 51. The conduit connector 52a of the general connector portion 52 at the proximal end of the tube unit 19 is detachably connected to the conduit connector 51a.

Further, the air/water supply conduit 60a is connected to the air/water supply conduit 60b that is passed through the inside of the tube unit 19, and the suction conduit 61a is connected to the suction conduit 61 b that is passed through the inside of the tube unit 19 and is branched inside the conduit connector 52a to open to the outside and link with an insertion port (also referred to as a "forceps port") 62 that enables insertion of a treatment instrument such as a forceps. This forceps port 62 is closed with a forceps plug 62a when it is not in use.

The rear ends of the air/water supply conduit 60b and the suction conduit 61b provide an air/water supply base 63 and a suction base 64 in the scope connector 41A.

Although not shown in the drawings, the air/water supply base 63 and the suction base 64 are connected to the air/water supply base and the suction base of the adaptor 42, respectively. Inside the adaptor 42, the air/water supply base is branched off into an air supply conduit and a water supply conduit.

As shown in Fig. 4, an electric contact point (connector) 111a is provided at the adaptor attaching portion in the endoscope system controlling device 5. Through an electric contact point 111b on the rear surface side of the adaptor 42, an electrical connector 43 that is provided on the front surface thereof is electrically connected to the electric contact point 111a. The electric contact point 111a includes a first connection portion for receiving a signal from the CCD 25 serving as an image pickup apparatus, and a second connection portion for receiving a signal from the UPD coils 58. In Fig. 4, reference numerals 111a and 111b are represented by reference numeral 111. Further, the electrical connector 43 is provided on the front surface of the adaptor 42 according to the present embodiment.

According to the present embodiment, an individual electric contact point connector 112 that is different from the electrical connector 43 is provided on the front surface of the adaptor 42 in a state in which a separating wall section (protrusion) 113a as electric shielding means is provided between the electrical connector 43 and the electric contact point connector 112.

An air supply connector 44a, a water supply connector 44b, and an auxiliary water supply connector 44c are provided on the front surface of the adaptor, adjacent to the electric contact point connector 112. These connectors are connected to a connector portion 114 of the AWS unit 4 at the rear surface side of the adaptor 42, and are respectively connected with an air supply conduit 4a, a water supply conduit 4b, and an auxiliary water supply conduit 4c via the connector portion 114. In Fig. 4, the air supply connector 44a, the water supply connector 44b, and the auxiliary water supply connector 44c are represented by reference numeral 44.

The air supply conduit 4a is connected to a water supply tank 48a, and the water supply conduit 4b is connected via an electromagnetic valve B1 to an air/water supply pump 65a and also connected via an electromagnetic valve B2 to the water supply tank 48a.

The auxiliary water supply conduit 4c is connected to an auxiliary water supply tank 48b that connects with an auxiliary water supply pump 65b.

On the front surface of the adaptor 42, a suction connector 145 is provided via the separating wall section 113b provided adjacent to the air supply connector 44a, the water supply connector 44b, and the auxiliary water supply connector 44c. The suction connector 145 is connected on the rear surface side thereof to a suction tank 48c that connects with a suction pump 65c through a suction conduit 4d inside the AWS unit 4.

Thus, in the present embodiment, by using the separating wall section 113b to separate or shield the air supply connector 44a, the water supply connector 44b, and the auxiliary water supply connector 44c that belong to a clean area from the suction connector 145 side that belongs to an unclean area, even if body fluids or the like spill out from the suction connector 145 when the scope connector 41I is detached, the air supply connector 44a and the like of the clean area side that are shielded by the separating wall section 113b are not affected.

The air/water supply pump 65a, the auxiliary water supply pump 65b, the suction pump 65c, and the electromagnetic valves B1 and B2 are connected to the AWS controlling unit 66 through a control line (drive line), and the opening and closing thereof are controlled by the AWS controlling unit 66 to thereby enable suction, air supply and water supply to be carried out.

The operation portion 22 of the endoscope main unit 18 is provided with a grasping portion 68 for the surgeon to grasp. In the present embodiment, as shown in Fig. 3A to Fig. 3D, the grasping portion 68 is formed of, for example, a side portion of a cylindrical element in the vicinity of the rear end (proximal end) of the operation portion 22 (opposite side to the insertion portion 21 side).

In this grasping portion 68, for example, three scope switches SW1, SW2, and SW3 which perform remote control operations such as releasing and freezing are provided along the longitudinal axis in the peripheral portion including the grasping portion 68 and are connected to each control circuit 57 (see Fig. 2) respectively.

Moreover, the proximal end surface (normally, as shown in Fig. 3, since the proximal end side is set in an upward direction when used for endoscopy, the proximal end surface is referred to as the upper end surface) provided at the rear end (proximal end) of the grasping portion 68 (or operation portion 22) is configured to be an inclined plane Sa. In the vicinity close to the opposite side from the position where the scope switches SW1, SW2, and SW3 are provided on the inclined plane Sa, a trackball 69 is provided which has a waterproof construction and which performs angle operations (bending operations) and the setting of other remote control operations by switching from the angle operations. The waterproof construction in this case is actually configured such that the encoder side for rotatably holding the trackball 69 or detecting the amount of rotation thereof is enclosed with a waterproof membrane and the trackball 69 is rotatably supported on the outside thereof.

A substantially U-shaped hook 70 that links the areas at the two ends in the longitudinal direction of the grasping portion 68 that is provided in the vicinity of the rear end of the operation portion 22. As shown in Fig. 3B, since the surgeon inserts fingers inside the hook 70 to grasp the operation portion 22 with the right hand (or left hand), it is possible to effectively prevent the endoscope 3A from falling down due to its own weight even in a case where the surgeon does not firmly grasp the grasping portion 68.

More specifically, the configuration is such that even if the endoscope 3A is going to fall down due to its own weight, the hook 70 stops at the hand there under and thus the endoscope 3A can be prevented from falling down. Thus, according to the present embodiment it is possible to effectively prevent the endoscope 3A from falling down due to its own weight even in a case where the surgeon does not firmly grasp (hold) the grasping portion 68. Accordingly, in a case in which the surgeon grasps the grasping portion 68 and performs various operations, if a hand or finger that grasps the grasping portion 68 becomes tired as a result of the operations, the endoscope 3A can be prevented from falling or the like by the surgeon inserting one portion of the hand inside the hook 70 even if the surgeon stops grasping (holding) the grasping portion 68, and thus operability can be improved.

Further, as shown in Fig. 3A to Fig. 3D, an air/water supply switch SW4 and a suction switch SW5 are symmetrically disposed on both sides of the trackball 69 on the inclined plane Sa.

The trackball 69 and the scope switches SW4 and SW5 are also connected to the control circuit 57. This situation will now be described further using Fig. 3A to Fig. 3D. In the front view shown in Fig. 3B, the operation portion 22 or the grasping portion 68 has a symmetrical shape with respect to a center line O (as a reference line) that extends in the longitudinal direction of the operation portion 22 or grasping portion 68. The trackball 69 is disposed on the inclined plane Sa at a position on the center line O. The air/water supply switch SW4 and suction switch SW5 are respectively disposed at right-left symmetrical positions on both sides of the trackball 69.

A back view that is the reverse side of this front view is shown in Fig. 3C. In this back view also, the operation portion 22 or the grasping portion 68 has a symmetrical shape with respect to the center line O, and three scope switches SW1, SW2, and SW3 are disposed on the outer surface of the grasping portion 68 along the center line O.

Further, in the present embodiment, as shown in Fig. 3A, the inclined plane Sa is formed with an angle ϕ as an obtuse angle that forms an angle greater than 90° with a line that is parallel with the center line O or a side surface of the grasping portion 68. In other words, the inclined plane Sa is formed in an inclined shape that forms an angle of θ with a surface perpendicular to the center line O of the grasping portion 68. At positions on the lower portion side of the inclined plane Sa, the trackball 69 and the air/water supply switch SW4 and suction switch SW5 are right-left symmetrically provided. As shown in Fig. 3B, the configuration enables easy operation of the trackball 69 or the like using the thumb of the hand that grasps the grasping portion 68.

The configuration in which operation means (instruction input section) such as the trackball 69 provided in the operation portion 22 are disposed so as to symmetrical with respect to the center line O in the longitudinal direction of the grasping portion 68 to enable appropriate operations in a case in which the surgeon grasps the operation portion 22 with either the right hand or the left hand is one feature of the endoscope 3A comprising the present endoscope system 1.

Further, in the grasping portion 68 is provided the hook 70 that links in a substantially U-shape the two ends, substantially, in the longitudinal direction of the grasping portion 68. Thus, even a state in which the surgeon insufficiently grasps the grasping portion 68 and the endoscope 3 is going to fall down due to its own weight, it is possible to realize a mechanism that can effectively prevent the endoscope 3 from falling down, since the surgeon's index finger or the like is inserted inside the hook 70 and the hook 70 is controlled by the index finger or the like.

Further, in the present endoscope 3A, since a configuration is adopted in which the grasping portion 68 is formed in the vicinity of the rear end of the operation portion 22 and a connection portion with the tube unit 19 is provided at a position closer to the insertion portion 21 than the position of the grasping portion 68, it is possible to reduce decentering of the position of the center of gravity from the central axis when the surgeon grasps the grasping portion 68.

That is, although in the conventional case when the tube unit 19 extends laterally from a further position to the rearward side (upper side) than that of the grasping portion, the position of the center of gravity in the case easily becomes off center due to the weight of the tube unit, in the present embodiment since the tube unit 19 is extended laterally from a position more on the insertion portion 21 side than the grasping portion 68, i.e. a lower side position, the eccentric amount of the center of gravity position can be reduced and operability can be improved.

Further, in the endoscope 3A, when an operator (user) such as a surgeon grasps the grasping portion 68 with the left hand or right hand, since the state is one in which the internal face side of the hook 70 is in a light contact with the vicinity of the side part of the index finger thereof, even if the center of gravity position is in a state in which it becomes off center and the central axis (i.e. the longitudinal direction of the operation portion 22) acts to incline, the hook 70 stops at the hand, and thus that inclination can be controlled and appropriate operability can be ensured.

As shown in Fig. 2, a power source line 71a and a signal line 71 b extending from the control circuit 57 are contactlessly and electrically connected to a power source line 73a and a signal line 73b inserted through the tube unit 19, via contactless transmitting sections 72a and 72b formed at the connector portion 51 and the general connector portion 52. The power source line 73a and the signal line 73b are connected to an electrical connector 74A at the scope connector 41A.

When a user connects the scope connector 41 A to the endoscope system controlling device 5 and the AWS unit 4, as shown in Fig. 4, the power source line 73a is connected via the electrical connector 43 for scope connection of the endoscope system controlling device 5 to a power supply unit 100 inside the endoscope system controlling device 5, and the signal line 73b is connected (via the power supply unit 100) to a UPD unit 76, a sending and receiving unit 101, a system controlling unit 117, and an image processing unit 116 as an endoscope signal processing section. The sending and receiving unit 101 is connected to an antenna section 101 a that sends and receives electric waves by radio transmission.

The contactless transmitting sections 72a and 72b are configured to form a transformer by electromagnetic coupling by placing respective pairs of coils adjacent to each other. That is, an end of the power source line 71a is connected to a coil forming the contactless transmitting section 72a, and an end of the other power source line 73a is also connected to a coil adjacent to the coil in the contactless transmitting section 72a.

Thus, an alternating current power that is transmitted by the power source line 73a passes through an electromagnetic coupling coil in the contactless transmitting section 72a so that power is transmitted to the power source line 71a side.

Further, an end of the signal line 71b is connected to a coil forming the contactless transmitting section 72b, and an end of the other signal line 73b is also connected to a coil adjacent to the coil in the contactless transmitting section 72b.

By forming a transformer by electromagnetic coupling, a signal is transmitted from the signal line 71b to the signal line 73b via the pair of coils, and a signal is also transmitted in the reverse direction.

Thus, another feature of the first endoscope 3A is that by adopting a configuration in which the endoscope main unit 18 can be contactlessly and detachably connected with the tube unit 19, even when washing or sterilization or the like are repeatedly performed it is possible to prevent the affects of corrosion and the like that occur at an electric contact point.

Further, by providing respective transparency sensors 243 at a halfway of the air/water supply conduit 60a and the suction conduit 61 a as shown in Fig. 2 and passing a light through the air/water supply conduit 60a and the suction conduit 61a that are respectively formed with a transparent tube, it is possible to detect the condition of dirt on the inner walls of the conduits or the transparency of fluids that pass through the inside of the conduits.

The transparency sensors 243 are connected to the control circuit 57 by signal lines.

Fig. 5 shows the configuration of the control circuit 57 and the like arranged inside the operation portion 22 of the endoscope main unit 18 in the endoscope 3A, and the configuration of the electrical system in major components disposed at each section of the insertion portion 21.

The CCD 25 and the LED 56 are disposed at the distal end portion 24 of the insertion portion 21 shown in the left bottom portion of Fig. 5, and at the bending portion 27 shown above the CCD 25 and the LED 56 in the figure, the angle actuator (in the present embodiment, specifically an EPAM) 27a and an encoder 27c are disposed. Also, in the flexible portion 53 shown above the bending portion 27 in the figure, a variable-rigidity actuator (in the present embodiment, specifically an EPAM) 54 and an encoder 54c are respectively disposed. Further, in the flexible portion 53, the transparency sensor 243 and the UPD coils 58 are disposed.

Furthermore, on the surface of the above described operation portion 22 above the flexible portion 53 of the insertion portion 21, the trackball 69, the air/water supply switch (SW4), the suction switch (SW5), and the scope switches (SW1 to SW3) are disposed. Further, as described later, operation of the trackball 69 is used to perform angular operations as well as the selection and setting of other functions.

As shown on the left side of Fig. 5, these are connected through signal lines with the control circuit 57 that includes the almost entire inside of the operation portion 22 as shown on the right side thereof. The control circuit 57 performs drive control of the functions of these components and signal processing and the like.

The control circuit 57 has a status managing section 81 comprising a CPU that manages the control states and the like. The status managing section 81 is connected to a status retaining memory 82 that retains (stores) the state of each portion and (according to the present embodiment) is also connected to a sending and receiving unit 83 of a wired system type that performs wire communication with the AWS unit 4.

Further, via an illumination controlling section 84 for controlling illumination, the status managing section 81 controls an LED driving section 85 which is controlled by the illumination controlling section 84. The LED driving section 85 applies a LED driving signal to the LED 56 to cause the LED 56 serving as illumination means to emit light.

An object such as a diseased part that is illuminated through the light emission by the LED 56 forms an image on the image pickup surface of the CCD 25 disposed at the image forming position through an unshown object lens which is attached to the observation window, and the image is subjected to photoelectric conversion by the CCD 25.

The CCD 25 outputs as an image pickup signal, signal charges which are accumulated through photoelectric conversion by the application of a CCD drive signal from the CCD driving section 86 controlled by the status managing section 81. This image pickup signal is converted from an analog signal to a digital signal through an A/D converter (abbreviated as ADC) 87, and is thereafter input to the status managing section 81, with the digital signal (image data) being stored in an image memory 88. The image data of the image memory 88 is sent to a data sending section 12' of the sending and receiving unit 83.

The image data is then sent to the endoscope system controlling device 5 side via the signal line 73b inside the tube unit 19 from the electrical connector 15.

As shown in Fig. 4, the image data that is sent to the endoscope system controlling device 5 is subjected to image processing by the image processing unit 116 to generate a video signal. The video signal is then output to the observation monitor 6 from the monitor connector 35 via the system controlling unit 117 that controls the entire endoscope system 1, to thereby display an endoscopic image on the display screen of the observation monitor 6.

In Fig. 4, the power supply unit 100 supplies power for operations to the UPD unit 76, the sending and receiving unit 101, the image processing unit 116, and the system controlling unit 117 and the AWS controlling unit 66 of the AWS unit 4.

The UPD unit 76 comprises an endoscope insertion shape calculating section that calculates the shape of the insertion portion 21 of the electronic endoscopes 3I based on insertion shape information from the plurality of UPD coils 58.

As shown in Fig. 5, an output signal of the above described ADC 87 is sent to a brightness detecting section 89, and information of the image brightness detected by the brightness detecting section 89 is sent to the status managing section 81. With this information, the status managing section 81 performs light adjustment to appropriately control the illumination light amount of the LED 56 via the illumination controlling section 84.

The status managing section 81 also controls an actuator driving section 92 via an angle controlling section 91 and controls driving of the angle actuator (EPAM) 27a by the actuator driving section 92. The driving amount of the angle actuator (EPAM) 27a is detected by the encoder 27c and controlled to match a value corresponding to the indicated value.

The status managing section 81 controls the actuator driving section 94 via a rigidity-variation controlling section 93, and controls driving of the variable-rigidity actuator (EPAM) 54 (in this case, one variable-rigidity actuator is shown as a representative of variable-rigidity actuators 54A and 54B) by the actuator driving section 94. The driving amount of the variable-rigidity actuator (EPAM) 54 is detected by the encoder 54c, and is controlled to match a value corresponding to the indicated value.

A detection signal from the transparency sensor 243 provided in the flexible portion 53 is converted to signal data corresponding to the transparency by a transparency detecting section 148, and is thereafter input to the status managing section 81. The status managing section 81 compares the input signal data with a transparency reference value that is previously stored in the status retaining memory 82 or the like. When the input signal data has reached the reference value, information to that effect is sent from the sending and receiving unit 83 through the AWS unit 4 to the endoscope system controlling device 5 side to display a message that the reference value has been reached on the observation monitor 6.

Further, an operation signal corresponding to the operating amount of the trackball 69 is input to the status managing section 81 from the trackball 69 and the like provided in the operation portion 22 via a trackball displacement detecting section 95.

Switch-pressing operations such as turning on the air/water supply switch, the suction switch, and the scope switch are detected by a switch-pressing detecting section 96, and the detected information is input to the status managing section 81.

The control circuit 57 comprises a power source transmitting and receiving section 97 and a power source generating section 98. The power source transmitting and receiving section 97 is, more specifically, the contactless transmitting section 72a at the operation portion 22, and is the electrical connector 74A at the distal end of the tube unit 19. Electric power transmitted by the power source generating section 98 is converted to DC electric power at the power source generating section 98. The electric power generated by the power source generating section 98 supplies each portion inside the control circuit 57 with electric power required for the operation thereof.

As described above, according to the present embodiment, in the endoscope system controlling device 5 and the AWS unit 4, a scope connection connector 40 to which the scope connectors 41I (I = A to D) of the endoscope are detachably connected is provided.

More specifically, in an endoscope system comprising the present embodiment, as shown in Fig. 4 the scope connector 41 A of the first endoscope is detachably connected, and as shown in Fig. 6 the scope connectors 41B to 41D (the scope connector 41A is also simultaneously shown) of the second to fourth endoscopes can also be similarly connected.

The first scope connector 41 A has the electrical connector 174A that is connected with the power source line 73a and the signal line 73b. The first scope connector 41 A also includes an air supply connector 63', a water supply connector 63b, and a suction connector 64 that are respectively provided at the ends of an air supply conduit 60b', a water supply conduit 59b, and a suction conduit 61 b.

The second scope connector 41B does not have the electrical connector 174A connecting with the electrical connector 43. Instead, the second scope connector 41B has an electric contact point connector 112b connecting with the electric contact point connector 112. Although the second scope connector 41B does not have the electrical connector 174A, it includes a dummy portion that is made in the shape of the electrical connector 174A.

Further, the third scope connector 41C is formed in a shape in which the dummy portion that is cut at a section denoted by the reference character P in the second scope connector 41B has been eliminated. The third scope connector 41C also has an electric contact point connector 112c in which the number of electric contact points is less than in the electric contact point connector 112b in the second scope connector 41B.

The fourth scope connector 41D is formed in the same shape as the third scope connector 41C, and is configured without an electric contact point.

The third scope connector 41C and the fourth scope connector 41D have an auxiliary water supply connector 115 in addition to the air supply connector 63', the water supply connector 63b, and the suction connector 64 that are in the first scope connector 41A.

According to the present embodiment, as shown in Fig. 4, the scope connection connector 40 is formed between the upper end vicinity on the outer surface of the AWS unit 4 and the lower end vicinity on the outer surface of the endoscope system controlling device 5. By attaching the adaptor 42 to a concave adaptor attaching portion forming the scope connection connector 40, it is possible to connect and use the scope connectors 41 A to 41 D of the first endoscope to fourth endoscope.

The structure on the front surface side of the adaptor 42 is configured to connect the electric contact point 111 b on the rear face side and the top end side to the electric contact point 111a provided on the endoscope system controlling device 5 side. By adopting this configuration, in the case of the scope connection connector 40 formed adjacent to both the AWS unit 4 and the endoscope system controlling device 5 also, the scope connectors 41 A to 41 D can be connected with one touch by inserting the adaptor 42 therebetween.

As described above, in the case of the present embodiment, a configuration is adopted in which the UPD unit 76 is provided on the endoscope system controlling device 5 side. The scope connector 41 A of the endoscope has a connector for inputting signals with insertion shape information of the UPD coils 58 and, together with other video signals and the like, can connect to the endoscope system controlling device 5 through the adaptor 42. Further, the AWS unit 4 is configured to receive a power supply from the power supply unit 100 inside the endoscope system controlling device 5 through a power source connector 75a.

Further, the AWS controlling unit 66 is connected to the system controlling unit 117 inside endoscope system controlling device 5E through a signal connector 66a.

According to the present embodiment, since separation means such as a separating wall section 113b is formed between a conduit connector belonging to a clean area and a connector belonging to an unclean area, attachment and detachment operations of the scope connectors 41 A to 41D are facilitated.

Further, because the UPD unit 76 is built in the endoscope system controlling device 5, the operations for attaching and detaching the scope connector 41A of the endoscope are facilitated since the signal connectors of the UPD coils 58 are integrated with another connector for video signals and the like.

Furthermore, space savings are realized since separate casings need not be provided. Since connectors need not be separately provided either, attachment and detachment can be carried out with one touch, and thus operability is also enhanced.

As described in the foregoing, according to the embodiment of the present invention there is an effect that a processing system that performs signal processing of image pickup signals of an electronic endoscope and a processing system that calculates an insertion shape of the endoscope are integrated, so that an operation to connect the electronic endoscope and a signal processing device can be simplified.

It should be understood that the present invention also includes embodiments and the like that are configured by modifying one part of the above described embodiment.

The present invention is not limited to the above described embodiment and various changes and modifications thereof are possible without departing from the spirit or scope of the invention.

This application claims priority from Japanese Patent Application No. 2005-276626 filed on Sept. 22, 2005, the entire contents of which are incorporated herein by this reference.

## Claims

1. An endoscope apparatus (1), comprising:
an electronic endoscope (3A) having a shape information generating section (58) for generating insertion shape information inside an insertion portion (21), and an image pickup section (25) that picks up images inside a body cavity; and
a signal processing device (5) integrally formed of an endoscope signal processing section (116) that drives the electronic endoscope (3A) and processes image pickup signals from the electronic endoscope (3A), and an endoscope insertion shape calculating section (76) that calculates an insertion shape of the electronic endoscope (3A) based on the insertion shape information from the shape information generating section (58); and
an endoscope peripheral device (4) having at least one of functions of air supply, water supply, and suction;
the endoscope apparatus (1) **characterized by** further comprising:
an adaptor (42); and
an adaptor attaching portion provided in the signal processing device (5) and which is used for attaching the adaptor (42);
the adaptor (42) including:
an electrical connection portion (43) that detachably connects a first electrical connector (74A, 174A) of the electronic endoscope (3A) that connects the shape information generating section (58) and the image pickup section (25) and a second electrical connector (111a) of the signal processing device (5); and
a conduit connection portion (44) adapted for detachably connecting and inserted between an endoscope side conduit connector (63) provided in a connector portion (41A) of the electronic endoscope (3A) and a peripheral device side conduit connector (114) provided in the endoscope peripheral device (4), **characterized in that**
the second electrical connector (111a) of the signal processing device (5) includes a first connection portion to be connected to the image pickup section (25) of the electronic endoscope (3A) and a second connection portion to be connected to the shape information generating section (58) of the electronic endoscope (3A), and
wherein the first and second connection portions are provided in the adaptor attaching portion for attaching the adaptor (42).

2. The endoscope apparatus according to claim 1, wherein the first and second connection portions include respective electric contact points.

3. The endoscope apparatus according to claim 1, wherein the conduit connection portion (44) includes a conduit for at least one of the air supply, water supply, or suction of the endoscope peripheral device (4).

4. The endoscope apparatus according to claim 1, wherein the endoscope peripheral device (4) has air supply, water supply, and suction functions.

5. The endoscope apparatus according to claim 4, wherein the conduit connection portion (44) includes three conduits for the air supply, water supply, and suction of the endoscope peripheral device (4), and the adaptor (42) is, on a side thereof, connected with the endoscope side conduit connector (114) and has a separation portion (113b) between two conduits (44) for the air supply and the water supply and a conduit (145) for the suction.

6. The endoscope apparatus according to claim 1, wherein the adaptor (42) comprises a connector (112) that is formed adjacent to the endoscope side conduit connector (63) in the electronic endoscope (3A), and is used for electrically connecting the first electrical connector (112b, 112c) of the electronic endoscope (3A) and another electrical connector of the adaptor attaching portion of the signal processing device (5).

7. The endoscope apparatus according to claim 6, wherein the adaptor (42) has, on a side connected with the first electrical connector (112b, 112c) of the electronic endoscope (3A), a separation portion (113a) between the first electrical connector (112) and the other electrical connection portion (43).

## Patentansprüche

1. Endoskopvorrichtung (1), aufweisend
ein elektronisches Endoskop (3A), das einen Forminformations-Erzeugungsbereich (58) zum Erzeugen von Informationen über die Einführform innerhalb eines Einführbereichs (21) und einen Bildaufnahmebereich (25), der Bilder im Innern einer Körperhöhle aufnimmt, aufweist; und
eine Signalverarbeitungsvorrichtung (5), die als integraler Bestandteil eines Endoskop-Signalverarbeitungsbereichs (116), welcher das elektronische Endoskop (3A) antreibt und Bildaufnahmesignale vom elektronischen Endoskop (3A) verarbeitet, und ein Endoskop-Einführformberechnungsbereich (76), der eine Einführform des elektronischen Endoskops (3A) auf der Basis der Einführforminformation vom Forminformations-Erzeugungsbereich (58) berechnet; und
ein Endoskop-Peripheriegerät (4), das zumindest eine der Funktionen wie Luftzufuhr, Wasserzufuhr und Absaugung aufweist;
wobei die Endoskopvorrichtung (1) **dadurch gekennzeichnet ist, dass** sie des Weiteren aufweist:
einen Adapter (42); und
einen Adapter-Anbringbereich, der in der Signalverarbeitungsvorrichtung (5) vorgesehen ist und zum Anbringen des Adapters (42) verwendet wird;
wobei der Adapter (42) enthält:
einen elektrischen Verbindungsbereich (43), der abnehmbar einen ersten elektrischen Anschluss (74A, 174A) des elektronischen Endoskops (3A), der den Forminformations-Erzeugungsbereich (58) und den Bildaufnahmebereich (25) verbindet, mit einem zweiten elektrischen Anschluss (111a) der Signalverarbeitungsvorrichtung (5) verbindet; und
einen Kanalverbindungsbereich (44), der abnehmbar einen Kanalanschluss (63) an der Endoskopseite, der in einem Anschlussbereich (41A) des elektronischen Endoskops (3A) vorgesehen ist, und einen Kanalanschluss (114) an der Peripheriegeräteseite, der im Endoskop-Peripheriegerät (4) vorgesehen ist, zu verbinden vermag und zwischen diesen eingefügt ist, **dadurch gekennzeichnet, dass**
der zweite elektrische Anschluss (111a) der Signalverarbeitungsvorrichtung (5) einen ersten Verbindungsbereich, der an den Bildaufnahmebereich (25) des elektronischen Endoskops (3A) anzuschließen ist, und einen zweiten Verbindungsbereich, der an den Forminformations-Erzeugungsbereich (58) des elektronischen Endoskops (3A) anzuschließen ist, enthält und
wobei der erste und der zweite Verbindungsbereich im Adapter-Anbringbereich zum Anbringen des Adapters (42) vorgesehen sind.

2. Endoskopvorrichtung nach Anspruch 1, wobei der erste und der zweite Verbindungsbereich entsprechende elektrische Kontaktpunkte enthalten.

3. Endoskopvorrichtung nach Anspruch 1, wobei der Kanalverbindungsbereich (44) einen Kanal für Luftzufuhr und/oder Wasserzufuhr und/oder Absaugung des Endoskop-Peripheriegeräts (4) enthält.

4. Endoskopvorrichtung nach Anspruch 1, wobei das Endoskop-Peripheriegerät (4) Funktionen für Luftzufuhr, Wasserzufuhr und Absaugung aufweist.

5. Endoskopvorrichtung nach Anspruch 4, wobei der Kanalverbindungsbereich (44) drei Kanäle für Luftzufuhr, Wasserzufuhr und Absaugung des Endoskop-Peripheriegeräts (4) enthält und der Adapter (42) an einer seiner Seiten mit dem Kanalanschluss (114) an der Endoskopseite verbunden ist und zwischen zwei Kanälen (44) für die Luftzufuhr und die Wasserzufuhr und einem Kanal (145) für die Absaugung einen Trennbereich (113b) aufweist.

6. Endoskopvorrichtung nach Anspruch 1, wobei der Adapter (42) einen Anschluss (112) umfasst, der angrenzend an den Kanalanschluss (63) an der Endoskopseite im elektronischen Endoskop (3A) ausgebildet ist und zum elektrischen Verbinden des ersten elektrischen Anschlusses (112b, 112c) des elektronischen Endoskops (3A) und eines weiteren elektrischen Anschlusses des Adapteranbringbereichs der Signalverarbeitungsvorrichtung (5) verwendet wird.

7. Endoskopvorrichtung nach Anspruch 6, wobei der Adapter (42) an einer Seite, die mit dem ersten elektrischen Anschluss (112b, 112c) des elektronischen Endoskops (3A) verbunden ist, zwischen dem ersten elektrischen Anschluss (112) und dem anderen elektrischen Verbindungsbereich (43) einen Trennbereich (113a) aufweist.

## Revendications

1. Appareil d'endoscope (1) comprenant :
un endoscope électronique (3A) comportant une section de génération d'information de forme (58) destinée à générer une information de forme d'insertion à l'intérieur d'une partie d'insertion (21), et une section de capture d'images (25) qui capture des images à l'intérieur d'une cavité de corps ; et
un dispositif de traitement de signal (5) intégralement formé d'une section de traitement de signal d'endoscope (116) qui attaque l'endoscope électronique (3A) et traite les signaux de capture d'images provenant de l'endoscope électronique (3A), et d'une section de calcul de forme d'insertion d'endoscope (76) qui calcule une forme d'insertion de l'endoscope électronique (3A) sur la base de l'information de forme d'insertion provenant de la section de génération d'information de forme (58) ; et
un dispositif périphérique d'endoscope (4) possédant au moins l'une parmi des fonctions d'alimentation en air, d'alimentation en eau et d'aspiration ;
l'appareil d'endoscope (1) étant **caractérisé en ce qu'**il comprend en outre :
un adaptateur (42) ; et
une partie de fixation d'adaptateur prévue dans le dispositif de traitement de signal (5) et qui est utilisée pour fixer l'adaptateur (42) ;
l'adaptateur (42) incluant :
une partie de connexion électrique (43) qui connecte de manière détachable un premier connecteur électrique (74A, 174A) de l'endoscope électronique (3A) qui connecte la section de génération d'information de forme (50) et la section de capture d'images (25) et un deuxième connecteur électrique (111a) du dispositif de traitement de signal (5) ; et
une partie de connexion de conduit (44) adaptée pour être connectée de manière détachable et insérée entre un connecteur de conduit côté endoscope (63) prévu dans une partie de connecteur (41A) de l'endoscope électronique (3A) et un connecteur de conduit côté dispositif périphérique (114) prévu dans le dispositif périphérique d'endoscope (4),
**caractérisé en ce que**
le deuxième connecteur électrique (111a) du dispositif de traitement de signal (5) comprend une première partie de connexion devant être connectée à la section de capture d'images (25) de l'endoscope électronique (3A) et une deuxième partie de connexion devant être connectée à la section de génération d'information de forme (58) de l'endoscope électronique (3A), et
dans lequel les première et deuxième parties de connexion sont prévues dans la partie de fixation d'adaptateur pour fixer l'adaptateur (42).

2. Appareil d'endoscope selon la revendication 1, dans lequel les première et deuxième parties de connexion comprennent des points de contact électrique respectifs.

3. Appareil d'endoscope selon la revendication 1, dans lequel la partie de connexion de conduit (44) comprend un conduit pour au moins une fonction parmi l'alimentation en air, l'alimentation en eau, ou l'aspiration du dispositif périphérique d'endoscope (4).

4. Appareil d'endoscope selon la revendication 1, dans lequel le dispositif périphérique d'endoscope (4) possède des fonctions d'alimentation en air, d'alimentation en eau, et d'aspiration.

5. Appareil d'endoscope selon la revendication 4, dans lequel la partie de connexion de conduit (44) comprend trois conduits destinés à l'alimentation en air, l'alimentation en eau, et l'aspiration du dispositif périphérique d'endoscope (4), et l'adaptateur (42) est, sur un côté de celui-ci, connecté au connecteur de conduit côté endoscope (114) et possède une partie de séparation (113b) entre deux conduits (44) pour l'alimentation en air et l'alimentation en eau et un conduit (145) pour l'aspiration.

6. Appareil d'endoscope selon la revendication 1, dans lequel l'adaptateur (42) comprend un connecteur (112) qui est formé adjacent par rapport au connecteur de conduit côté endoscope (63) dans l'endoscope électronique (3A), et est utilisé pour connecter électriquement le premier connecteur électrique (112b, 112c) de l'endoscope électronique (3A) et un autre connecteur électrique de la partie de fixation d'adaptateur du dispositif de traitement de signal (5).

7. Appareil d'endoscope selon la revendication 6, dans lequel l'adaptateur (42) possède, sur un côté connecté avec le premier connecteur électrique (112b, 112c) de l'endoscope électronique (3A), une partie de séparation (113a) entre le premier connecteur électrique (112) et l'autre partie de connexion électrique (43).
